# EUROPÄISCHE PATENTSCHRIFT

(11) **EP 0 440 913 B1**
(45) Veröffentlichungstag und Bekanntmachung des Hinweises auf die Patenterteilung: **21.02.1996**
(21) Anmeldenummer: 90123199.3
(22) Anmeldetag: 04.12.1990
(51) Int. Cl.: C07C 51/46, C07C 51/14

(54) **Verfahren zur Herstellung von tertiären Carbonsäuren**
Process for preparing tertiary carboxylic acids
Procédé de préparation d'acides carboxyliques tertiaires

(30) Priorität: 03.02.1990 DE 4003232
(43) Veröffentlichungstag der Anmeldung: 14.08.1991
(73) Patentinhaber: HÜLS AKTIENGESELLSCHAFT, D-45764 Marl (DE)
(72) Erfinder: Müller, Wolfgang Hans Eduard, Dr., W-4370 Marl (DE); Hartmann, Manfred, W-4370 Marl (DE)

(56) Entgegenhaltungen:
- EP-A- 0 125 575
- DE-A- 1 468 397
- DE-B- 1 035 656

## Beschreibung

Die Erfindung betrifft ein Verfahren zur Herstellung von tertiären Carbonsäuren aus Kohlenmonoxid, den entsprechenden Olefinen und Wasser an einem sauren Katalysator.

Tertiäre Carbonsäuren lassen sich nach der sogenannten "Koch-Synthese" aus den entsprechenden Olefinen, Kohlenmonoxid und Wasser herstellen. So erhält man z. B. aus Isobuten die 2,2-Dimethyl-propansäure: 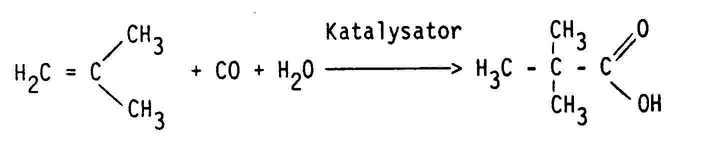
Als Katalysatoren werden hauptsächlich starke Protonensäuren, wie z. B. Schwefelsäure, Phosphorsäure und Fluorwasserstoffsäure, oder Lewis-Säuren, wie z. B. Bortrifluorid, Aluminiumchlorid und Antimon(V)-chlorid verwendet (siehe "Methoden der organischen Chemie (Houben-Weyl)", 4. Auflage, Band E5, 1985, S. 315).

Es ist bekannt, zur Abtrennung der bei der "Koch-Synthese" anfallenden Nebenprodukte das Reaktionsgemisch vor der Reindestillation der tertiären Carbonsäuren mit Wasser (Hydrocarbon Processing, Vol. 43, Nr. 11, November 1964, S. 186/187) sowie mit Schwefelsäure, Natriumhydrogenkarbonat und Zitronensäure zu waschen (Ullmanns Encyklopädie der technischen Chemie, 4. Auflage, Band 9, S. 140 und J. Falbe: New Syntheses with Carbonmonoxide (1980), S. 407).

Gemäß JP-AS 73/16 897 wird das die tertiären Carbonsäuren enthaltende Rohprodukt vor der Reindestillation mit einem oxidierenden Agens, wie z. B. mit Luft oder Sauerstoff, behandelt und gemäß US-Defensive 864 004 katalytisch hydriert.

DE-OS 15 68 391 lehrt ein Verfahren zur Herstellung von Neosäuren nach der "Koch-Synthese", wobei man die gebildeten Neosäuren durch Extraktion mit Cyclohexan und wäßrigem Kaliumhydroxid aus dem Reaktionsprodukt gewinnt (siehe Beispiel 2).

Auch gemäß US-PS 3 489 779 und US-PS 3 151 139 werden die gebildeten Neosäuren durch Extraktion aus dem Reaktionsgemisch abgetrennt, und zwar mit Heptan als Extraktionsmittel bzw. mit wäßriger Ammoniak-Lösung in Gegenwart eines mit Wasser nicht mischbaren organischen Lösemittels, das die Verunreinigungen enthält.

JP-AS 71/35 724 offenbart ein Verfahren zur Gewinnung von Trimethylessigsäure, wobei aus dem bei der "Koch-Synthese" erhaltenen Rohprodukt dimeres und trimeres Isobuten als azeotrope Gemische mit Wasser und n-Amylalkohol abdestilliert werden.

Gemäß JP-AS 73/00 807 werden aus dem Trimethylessigsäure-Rohprodukt der "Koch-Synthese" als Verunreinigungen dimeres Isobuten mit Wasser als Schleppmittel und trimeres Isobuten mit Wasser und Trimethylessigsäure als Schleppmittel durch Azeotropdestillation entfernt.

Die Verfahren gemäß dem Stand der Technik zur Gewinnung von tertiären Carbonsäuren aus dem Rohprodukt der "Koch-Synthese" sind aufwendig und schwierig durchzuführen, da insbesondere bei der Flüssigphasen-Flüssigphasen-Extraktion kleine Mengen von Nebenbestandteilen oder Verunreinigungen zu Störungen des Betriebsablaufs führen können.

Die Aufarbeitungsverfahren gemäß JP-AS 71/35 724 und JP-AS 73/00 807 eignen sich ausschließlich zur Isolierung von Trimethylessigsäure, wobei als Verunreinigungen sich nur dimeres und trimeres Isobuten azeotrop aus dem Trimethylessigsäure-Rohprodukt abdestillieren lassen.

Der Erfindung liegt daher die Aufgabe zugrunde, ein wirtschaftlicheres und leichter durchführbares Verfahren zur Herstellung von tertiären Carbonsäuren nach der "Koch-Synthese" zu entwickeln, in welchem sich die gebildeten tertiären Carbonsäuren möglichst frei von anderen Stoffen auf einfache und wirkungsvolle Weise aus dem Reaktionsgemisch gewinnen lassen.

Es wurde nun überraschenderweise gefunden, daß sich die Nebenprodukte aus dem Rohprodukt der "Koch-Synthese" auf einfache und elegante Weise durch eine Azeotroprektifikation mit Alkandiolen als Schleppmittel entfernen und sich so die tertiären Carbonsäuren in reiner Form gewinnen lassen.

Gegenstand der vorliegenden Erfindung ist daher ein Verfahren zur Herstellung von tertiären Carbonsäuren aus Kohlenmonoxid, den entsprechenden Olefinen und Wasser an einem sauren Katalysator, das dadurch gekennzeichnet ist, daß die Nebenprodukte aus dem Reaktionsmisch durch Azeotroprektifikation mit Alkandiolen als Schleppmittel nach Abtrennung des Katalysators entfernt und die tertiären Carbonsäuren gewonnen werden.

Die Herstellung des die tertiären Carbonsäuren enthaltenden Reaktionsgemisches erfolgt nach der "Koch-Synthese" gemäß dem Stand der Technik.

Bevorzugt läßt sich das erfindungsgemäße Verfahren anwenden, wenn die Nebenprodukte ähnliche Siedepunkte wie die hergestellten tertiären Carbonsäuren aufweisen, d. h. die Nebenprodukte, die ähnliche Siedepunkte wie die hergestellten tertiären Carbonsäuren aufweisen, werden aus dem Reaktionsgemisch durch Azeotroprektifikation mit Alkandiolen als Schleppmittel nach Abtrennung des Katalysators entfernt und die tertiären Carbonsäuren gewonnen.

Bekanntlich lassen sich nämlich die Nebenprodukte, die im gleichen Siedebereich wie die hergestellten tertiären Carbonsäuren sieden, von diesen nicht durch einfache Rektifikation trennen.

Eine bevorzugte Variante des erfindungsgemäßen Verfahrens ist es, daß vor der Azeotroprektifikation mit den Alkandiolen als Schleppmittel die nicht umgesetzten Einsatzolefine aus dem Reaktionsgemisch abgetrennt werden, besonders bevorzugt werden die nicht umgesetzten Einsatzolefine nach Abtrennung aus dem Reaktionsgemisch in den Reaktionsteil zurückgeführt.

Bevorzugt kann man das erfindungsgemäße Verfahren zur Herstellung von tertiären Carbonsäuren mit 6 bis 13 Kohlenstoffatomen, besonders bevorzugt zur Herstellung von tertiären Nonansäuren und Dekansäuren einsetzen.

Bei der Herstellung von tertiären Carbonsäuren nach der "Koch-Synthese" enthält das Rohprodukt erhebliche Anteile an Nebenprodukten. Unter Nebenprodukten sind z. B. zu verstehen:
Olefine und Paraffine aus den Einsatzstoffen und durch chemische Reaktionen gebildete Alkohole, Ester und Carbonylverbindungen sowie Oligomerisierungs-, insbesondere Dimerisierungs- und Trimerisierungsprodukte der eingesetzten Olefine und eine Fülle weiterer Stoffe, die durch Auf- bzw. Abbau der Kohlenstoffkette der Olefine mit allen Folgereaktionen entstehen können.

Das erfindungsgemäße Verfahren eignet sich bevorzugt, um bei der Herstellung von tertiären Carbonsäuren als Nebenprodukte gebildete Oligomere der Einsatzolefine und/oder Alkohole und/oder Ester und/oder Carbonylverbindungen und/oder aus den Einsatzstoffen stammende Paraffine und/oder Olefine durch Azeotroprektifikation mit Alkandiolen als Schleppmittel zu entfernen und so die tertiären Carbonsäuren zu gewinnen.

Vorzugsweise werden bei der Azeotroprektifikation als Schleppmittel Alkandiole eingesetzt, die mit den bei der Herstellung von tertiären Carbonsäuren als Nebenprodukte gebildeten Oligomeren der Einsatzolefine und/oder Alkoholen und/oder Estern und/oder Carbonylverbindungen und/oder aus den Einsatzstoffen stammenden Paraffinen und/oder Olefinen Heteroazeotrope bilden.

Alkandiole, die mit den erfindungsgemäß abzutrennenden Nebenprodukten keine Heteroazeotrope, sondern Homoazeotrope bilden, wie z. B. 2-Methylpentandiol-2,4 bei der Herstellung von tertiären Nonan- und Dekansäuren, sind als Schleppmittel ebenfalls geeignet.

Die erfindungsgemäß angewandte Azeotroprektifikation mit Alkandiolen als Schleppmittel kann sowohl diskontinuierlich als auch kontinuierlich durchgeführt werden.

Vorzugsweise wird die Azeotroprektifikation mit Alkandiolen als Schleppmittel unter gegenüber Atmosphärendruck vermindertem Druck durchgeführt.

Zur Herstellung von tertiären Nonansäuren und/oder tertiären Dekansäuren werden vorzugsweise bei der Azeotroprektifikation als Schleppmittel Alkandiole mit Siedepunkten unter Normaldruck von < 230 °C eingesetzt, wie z. B.
Propandiol-1,2,
Butandiol-1,2,
Butandiol-1,3,
Butandiol-2,3,
Pentandiol-1,2,
Pentandiol-2,4,
Hexandiol-1,2,
Hexandiol-2,5 und
2,2-Dimethylpropandiol-1,3,
besonders vorzugsweise wird Butandiol-1,3 als Schleppmittel bei der Azeotroprektifikation zur Herstellung von tertiären Nonansäuren und/oder tertiären Dekansäuren eingesetzt.

Alkandiole mit Siedepunkten unter Normaldruck von > 230 °C bilden bei der Herstellung von tertiären Nonan- und/oder Dekansäuren ebenfalls mit den Nebenprodukten Heteroazeotrope und eignen sich auch für die Abtrennung der Nebenprodukte aus dem Reaktionsgemisch.

Im Anschluß an die Azeotroprektifikation mit Alkandiolen als Schleppmittel werden die tertiären Carbonsäuren, z. B. durch Reindestillation, gewonnen.

Das erfindungsgemäße Verfahren bietet gegenüber den Verfahren gemäß dem Stand der Technik wesentliche Vorteile durch Störunanfälligkeit, höhere Produktreinheit und durch den Einsatz einfacherer technischer Apparaturen.

Die Erfindung wird durch die folgenden Beispiele näher erläutert:

### Beispiel 1

In einer Rektifikationskolonne mit einer Sulzer-Laborpackung von 1 m Länge und einer Nennweite von 50 mm werden 2 000 g vom Katalysator und von Leichtsiedern (wie z. B. Tripropen) befreite Rohneodekansäure mit 2 000 g Butandiol-1,3 als Schleppmittel bei einem Druck am Kolonnenkopf von 4 mbar und einem Rücklaufverhältnis von 5 diskontinuierlich rektifiziert. Die Rohneodekansäure wird in bekannter Weise nach der "Koch-Synthese" aus Tripropen, Kohlenmonoxid und Wasser mit Bortrifluorid, Wasser und Kupfer(I)oxid als Katalysator bei einem Druck von 10 bar und einer Temperatur von 30 °C hergestellt. Gemäß gaschromatographischer Analyse ergibt sich für die so erhaltene Rohneodekansäure die folgende Zusammensetzung:
76 Gew.-% Dekansäuren
23 Gew.-% C₁₈-Kohlenwasserstoffe
1 Gew.-% sonstige Stoffe

Bei der Rektifikation der Rohneodekansäure werden die folgenden Fraktionen folgender Zusammensetzungen, zum Teil unter Phasentrennung (als Heteroazeotrope), bei den folgenden Siedepunkten unter einem Druck von 4 mbar abs. abgezogen:
Fraktion 1 (Kp₄ ≦ 85 °C), obere Phase (156 g):
< 0,1 Gew.-% Butandiol-1,3
< 0,1 Gew.-% Dekansäuren
83,3 Gew.-% C₁₈-Kohlenwasserstoffe
16,7 Gew.-% sonstige Stoffe, wie z. B. andere Kohlenwasserstoffe, Ester und Carbonsäuren mit weniger als 10 C-Atomen.
Fraktion 1, untere Phase (233 g):
87,7 Gew.-% Butandiol-1,3
< 0,1 Gew.-% Dekansäuren
0,3 Gew.-% C₁₈-Kohlenwasserstoffe
12,0 Gew.-% sonstige Stoffe, wie z. B. Ester und Carbonsäuren mit weniger als 10 C-Atomen
Fraktionen 2 bis 5 (Kp₄ = 85 °C), obere Phasen (zusammen 313 g):
< 0,1 Gew.-% Butandiol-1,3
< 0,1 Gew.-% Dekansäuren
100,0 Gew.-% C₁₈-Kohlenwasserstoffe
< 0,1 Gew.-% sonstige Stoffe
Fraktionen 2 bis 5, untere Phasen (zusammen 1 293 g):
99,3 Gew.-% Butandiol-1,3
0,5 Gew.-% Dekansäuren
0,2 Gew. -% C₁₈-Kohlenwasserstoffe
< 0,1 Gew.-% sonstige Stoffe

Ab der 6. Fraktion fiel ein homogenes Destillat an:
Fraktionen 6 bis 9 (Kp₄ = 86 - 115 °C) (zusammen 882 g):
Mischungen von Butandiol-1,3 und Dekansäuren
Fraktionen 10 bis 12 (Kp₄ = 115 - 120 °C) (zusammen 625 g):
> 99,9 Gew.-% Dekansäuren
498 g Destillationsrückstand:
84,8 Gew.-% Dekansäuren
15,2 Gew.-% sonstige Stoffe (Hochsieder)

### Beispiel 2 (Vergleichsbeispiel)

Führt man die Rektifikation von Rohneodekansäure wie im Beispiel 1 durch, allerdings ohne Butandiol-1,3 als Schleppmittel, so ergibt sich nur eine maximale Anreicherung der C₁₈-Kohlenwasserstoffe auf 38,0 Gew.-% und eine maximale Anreicherung der Dekansäuren auf 98,3 Gew.-%.

### Beispiel 3 (Vergleichsbeispiel)

Wird Beispiel 2 wiederholt, jedoch mit einem von 5 auf 25 erhöhten Rücklaufverhältnis, so erreicht man eine maximale Anreicherung der C₁₈-Kohlenwasserstoffe auf 47,6 Gew.-% und eine maximale Anreicherung der Dekansäuren auf 98,6 Gew.-%.

### Beispiel 4

Analog zu Beispiel 1 wird die Rohneodekansäure zusammen mit 1 000 g Butandiol-1,4 als Schleppmittel diskontinuierlich rektifiziert: Zuerst geht bei ca. 100 °C ein Gemisch (1 174 g) über, das sich in zwei flüssige Phasen trennt, wobei die leichteren Phasen (zusammen 587 g) ca. 25 Gew.-% Dekansäuren und ca. 71 Gew.-% C₁₈-Kohlenwasserstoffe sowie 0,5 Gew.-% Butandiol-1,4 und die schwereren Phasen (zusammen 587 g) 65 Gew.-% Butandiol-1,4, 27 Gew.-% Dekansäuren und 5 Gew.-% C₁₈-Kohlenwasserstoffe neben geringen Mengen an sonstigen Stoffen, wie z. B. anderen Kohlenwasserstoffen, enthalten. Anschließend wird bei 101 bis 106 °C ein binäres Azeotrop (1 027 g) mit 60 Gew.-% Butandiol-1,4 und 40 Gew.-% Dekansäuren und < 0,4 Gew.-% C₁₈-Kohlenwasserstoffen abgezogen, und danach geht nach einer Übergangsfraktion eine Fraktion (530 g) mit einem Gehalt von > 99,9 Gew.-% an Dekansäuren über.

### Beispiel 5

Es wird Beispiel 1 wiederholt, jedoch mit 1 182 g Propandiol-1,2 statt 2 000 g Butandiol-1,3 als Schleppmittel:
Die leichteren Phasen (zusammen 415 g) der bei ca. 71 °C übergehenden Fraktionen, die sich nach Kondensation in zwei flüssige Phasen trennen, enthalten 99,8 bis 99 Gew.-% C₁₈-Kohlenwasserstoffe und 0,2 bis 1,0 Gew.-% sonstige Stoffe, wie z. B. andere Kohlenwasserstoffe. Die schwereren Phasen (zusammen 4 181 g) setzen sich aus 99,8 Gew.-% Propandiol-1,2 und 0,2 Gew.-% C₁₈-Kohlenwasserstoffen zusammen. Sie werden größtenteils der Kolonne als zusätzlicher Rücklauf wieder zugeleitet. Nach einer Übergangsfraktion wird bei 119 bis 121 °C 100,0 Gew.-%ige Dekansäure (1 239 g) abgezogen.

### Beispiel 6

Beispiel 1 wird wiederholt, jedoch mit 1 118 g Butandiol-2,3 statt 2 000 g Butandiol-1,3 als Schleppmittel.

Die bei 63 bis 65 °C übergehenden Fraktionen trennen sich auch hier in zwei flüssige Phasen. Die leichteren Phasen (zusammen 385 g) enthalten 99,8 Gew.-% C₁₈-Kohlenwasserstoffe und 0,2 Gew.-% Butandiol-2,3, die schwereren Phasen (zusammen 7 484 g), die größtenteils in die Kolonne zurückgeführt werden, 98,3 Gew.-% Butandiol-2,3, 1 Gew.-% C₁₈-Kohlenwasserstoffe und 0,7 Gew.-% sonstige Stoffe, wie z. B. Carbonsäuren mit weniger als 10 C-Atomen. Nach einer Übergangsfraktion geht dann bei 119 bis 121 °C Dekansäure (1 178 g) mit einem Gehalt von > 99,5 Gew.-% und mit einem Gehalt von < 0,2 Gew.-% Butandiol-2,3 und < 0,3 Gew.-% C₁₈-Kohlenwasserstoffen über.

### Beispiel 7

Es wird Beispiel 1 wiederholt, jedoch mit 1 063 g Butandiol-1,2 statt 2 000 g Butandiol-1,3 als Schleppmittel.

Die bei 73 bis 75 °C übergehende Fraktion trennt sich auch hier in zwei flüssige Phasen. Die leichtere Phase (388 g) enthält mehr als 99,5 Gew.-% C₁₈-Kohlenwasserstoffe, weniger als 0,3 Gew.-% Dekansäuren und weniger als 0,2 Gew.-% Butandiol-1,2. Die zugehörigen schwereren Phasen werden der Rektifikationskolonne wieder zugeleitet. Nach einer Übergangsfraktion fallen Fraktionen mit einem Gehalt von 99,5 bis 100,0 Gew.-% Dekansäuren (1 250 g) an.

### Beispiel 8

Beispiel 1 wird wiederholt, jedoch mit 160 g Pentandiol-1,5 statt 2 000 g Butandiol-1,3 als Schleppmittel. Die Rektifikation erfolgt bei einem Druck von 1 mbar abs.

Die zuerst bei 87 bis 90 °C übergehenden Fraktionen trennen sich auch hier in zwei flüssige Phasen. Die leichteren Phasen (zusammen 601 g) enthalten 72 Gew.-% C₁₈-Kohlenwasserstoffe neben 23 Gew.-% Dekansäuren, < 5 Gew.-% sonstige Stoffe, wie z. B. Carbonsäuren mit weniger als 10 C-Atomen, und < 1 Gew.-% Pentandiol-1,5. Die schwereren Phasen werden der Kolonne wieder zugeleitet. Anschließend wird nach einer Übergangsfraktion ein binäres Azeotrop (198 g) mit 30 Gew.-% Pentandiol-1,5 und 70 Gew.-% Dekansäuren abgezogen, und danach fallen nach einer Übergangsfraktion Dekansäuren (800 g) mit einem Gehalt von > 99,5 Gew.-% an.

### Beispiel 9

Beispiel 1 wird wiederholt, jedoch mit 550 g Pentandiol-2,4 statt 2 000 g Butandiol-1,3 als Schleppmittel. Die Rektifikation erfolgt bei einem Druck von 1 mbar abs.

Die bei 57 bis 68 °C übergehenden Fraktionen trennen sich auch hier in zwei flüssige Phasen. Die leichteren Phasen (zusammen 448 g) enthalten 99,8 Gew.-% C₁₈-Kohlenwasserstoffe und 0,2 Gew.-% Pentandiol-2,4. Die schwereren Phasen werden auch hier wieder in die Kolonne zurückgeführt. Die nach der Übergangsfraktion anfallenden Dekansäurefraktionen (zusammen 1 080 g) haben die folgende Zusammensetzung: > 99,5 Gew.-% Dekansäuren, < 0,3 Gew.-% C₁₈-Kohlenwasserstoffe und < 0,2 Gew.-% Pentandiol-2,4.

### Beispiel 10

Es wird Beispiel 1 wiederholt, jedoch mit 1 667 g Pentandiol-1,2 statt 2 000 g Butandiol-1,3 als Schleppmittel. Die Rektifikation erfolgt bei einem Druck von 1 mbar abs.

Die bei 68 °C übergehende Fraktion trennt sich auch hier in zwei flüssige Phasen. Die schwereren Phasen werden in die Kolonne zurückgeführt. Die leichtere Phase (403 g) enthält 99,6 Gew.-% C₁₈-Kohlenwasserstoffe, 0,2 Gew.-% Pentandiol-1,2 und 0,2 Gew.-% sonstige Stoffe, wie z. B. andere Kohlenwasserstoffe. Die nach der Übergangsfraktion anfallenden Dekansäurefraktionen (zusammen 1 246 g) haben einen Gehalt von > 99,5 Gew.-%.

### Beispiel 11

Es wird Beispiel 1 wiederholt, jedoch mit 1 000 g Rohneodekansäure und mit 2 800 g 2-Methylpentandiol-2,4 statt 2 000 g Butandiol-1,3 als Schleppmittel. Die Rektifikation erfolgt bei einem Druck von 1 mbar abs.

Das Destillat (2 913 g) mit einem Siedepunkt von 63 °C trennt sich nicht in zwei flüssige Phasen. Es enthält ca. 92 Gew.-% 2-Methylpentandiol-2,4 und 8 Gew.-% C₁₈-Kohlenwasserstoffe. Nach einer Übergangsfraktion geht eine Fraktion (90 g) mit ca. 80 Gew.-% Dekansäuren und 20 Gew.-% 2-Methylpentandiol-2,4 über, und nach einer weiteren Übergangsfraktion werden Dekansäuren (433 g) mit einem Gehalt von > 99,5 Gew.-% abgezogen.

### Beispiel 12

Beispiel 1 wird wiederholt, jedoch mit 895 g Hexandiol-2,5 statt 2 000 g Butandiol-1,3 als Schleppmittel. Die Rektifikation erfolgt bei einem Druck von 1 mbar abs.

Die bei 76 bis 82 °C übergehenden Fraktionen trennen sich in zwei flüssige Phasen. Die schwereren Phasen werden in die Kolonne zurückgeführt. Die leichteren Phasen (zusammen 371 g) enthalten 99,6 Gew.-% C₁₈-Kohlenwasserstoffe, 0,2 Gew.-% Hexandiol-2,5 und 0,2 Gew.-% sonstige Stoffe, wie z. B. Carbonsäuren mit weniger als 10 C-Atomen. Nach den Übergangsfraktionen werden Fraktionen mit 100,0 Gew.-% an Dekansäuren (846 g) erhalten.

### Beispiel 13

Beispiel 1 wird wiederholt, jedoch mit 1 102 g Hexandiol-1,2 statt 2 000 g Butandiol-1,3 als Schleppmittel. Die Rektifikation erfolgt bei einem Druck von 1 mbar abs.

Die bei 75 bis 76 °C übergehenden Fraktionen trennen sich auch hier in zwei flüssige Phasen. Die leichteren Phasen (zusammen 446 g) enthalten 98,5 Gew.-% C₁₈-Kohlenwasserstoffe, 1 Gew.-% Hexandiol-1,2 und 0,5 Gew.-% sonstige Stoffe, wie z. B. Carbonsäuren mit weniger als 10 C-Atomen. Nach mehreren Übergangsfraktionen fällt eine 99,8 Gew.-%ige Dekansäure (996 g) mit einem Gehalt von 0,2 Gew.-% C₁₈-Kohlenwasserstoffen an.

### Beispiel 14

Beispiel 1 wird wiederholt, jedoch mit 743 g Cyclohexandiol-1,2 statt 2 000 g Butandiol-1,3 als Schleppmittel. Die Rektifikation erfolgt bei einem Druck von 21 mbar abs.

Das Destillat trennt sich nicht in zwei flüssige Phasen. Nach Abtrennung einer Fraktion (214 g) mit
25 Gew.-% Cyclohexandiol-1,2,
2 Gew.-% Carbonsäuren mit weniger als 10 C-Atomen,
58 Gew.-% Kohlenwasserstoffen und
15 Gew.-% sonstigen Stoffen
gehen Fraktionen (zusammen 620 g) mit ca. 53 Gew.-% Cyclohexandiol-1,2 und ca. 47 Gew.-% C₁₈-Kohlenwasserstoffen über. Nach Übergangsfraktionen mit steigenden Mengen an Dekansäure wird schließlich eine Fraktion (851 g) mit einem Gehalt von > 99,5 Gew.-% an Dekansäure erhalten.

### Beispiel 15

Analog zu Beispiel 1 werden 2 000 g Rohneononansäure, die nach Abtrennung der C₈-Kohlenwasserstoffe 80 Gew.-% Nonansäure, 16 Gew.-% C₁₆-Kohlenwasserstoffe und 4 Gew.-% sonstige Stoffe, wie z. B. andere Kohlenwasserstoffe, andere Carbonsäuren und Ester, enthält, mit 750 g Butandiol-1,3 als Schleppmittel rektifiziert.

Die Rohneononansäure wird in bekannter Weise nach der "Koch-Synthese" aus Isookten, Kohlenmonoxid und Wasser mit Bortrifluorid, Wasser und Kupfer(I)oxid als Katalysator bei einem Druck von 10 bar und einer Temperatur von 30 °C hergestellt.

Die bei 65 bis 76 °C und einem Druck von 4 mbar abs. übergehende erste Fraktion (356 g) trennt sich in zwei flüssige Phasen, wobei sich die leichtere Phase (231 g) aus 10 Gew.-% Butandiol-1,3, 52 Gew.-% C₁₆-Kohlenwasserstoffen und 38 Gew.-% sonstigen Stoffen, wie z. B. anderen Kohlenwasserstoffen, zusammensetzt. Die weiteren Fraktionen, die bei 76 bis 88 °C abgezogen werden, trennen sich ebenfalls in zwei flüssige Phasen, deren leichtere Phasen (zusammen 195 g) 93 Gew.-% C₁₆-Kohlenwasserstoffe, 6 Gew.-% sonstige Stoffe, wie z. B. andere Kohlenwasserstoffe und Ester und 1 Gew.-% Butandiol-1,3 enthalten. Die schwereren Phasen werden der Rektifikationskolonne wieder zugeleitet. Nach mehreren Übergangsfraktionen wird Nonansäure (1 153 g) mit einer Reinheit von > 99,9 Gew.-% abgezogen.

### Beispiel 16

Beispiel 15 wird wiederholt, jedoch mit 1 174 g Propandiol-1,2 statt 750 g Butandiol-1,3 als Schleppmittel.

Die bei 48 bis 65 °C und bei einem Druck von 5 mbar abs. übergehende erste Fraktion trennt sich in zwei flüssige Phasen, wobei sich die leichtere Phase (123 g) aus 15 Gew.-% Propandiol-1,2, 15 Gew.-% C₁₆-Kohlenwasserstoffe und 70 Gew.-% sonstiger Stoffe, wie z. B. andere Kohlenwasserstoffe, zusammensetzt. Die weiteren Fraktionen, die bei 65 bis 75 °C abgezogen werden, trennen sich ebenfalls in zwei flüssige Phasen, deren leichtere Phasen (zusammen 301 g) 96 Gew.-% C₁₆-Kohlenwasserstoffe, 4 Gew.-% sonstige Stoffe, wie z. B. Ester und 0,3 Gew.-% Propandiol-1,2 enthalten. Nach mehreren Übergangsfraktionen wird Neononansäure (1 054 g) mit 99,8 Gew.-%iger Reinheit abgezogen.

### Beispiel 17

1,470 kg/h Rohneodekansäure werden nach Abtrennung des Katalysators und des nicht umgesetzten Nonens mit 0,795 kg/h Butandiol-1,3 als Schleppmittel in einem System, bestehend aus drei Kolonnen "A", "B" und "C", kontinuierlich rektifiziert.

In der Kolonne "A" werden die Nebenprodukte als azeotropes Gemisch mit Butandiol-1,3 abgetrennt und in Kolonne "B" die Zwischensieder, während in Kolonne "C" Neodekansäure als Reinprodukt über Kopf destilliert wird. Die Rohneodekansäure wird in bekannter Weise nach der "Koch-Synthese" aus Tripropen, Kohlenmonoxid und Wasser mit Bortrifluorid, Wasser und Kupfer(I)oxid als Katalysator bei einem Druck von 10 bar und einer Temperatur von 30 °C hergestellt.

Abb. 1 gibt das Fließschema des Systems aus den drei Kolonnen "A", "B" und "C" wieder.

Die hierzu gehörenden Verfahrensparameter aller drei Kolonnen sind der Tabelle 1 zu entnehmen.

Es werden die folgenden Mengenströme durchgesetzt:

| | | |
|---|---|---|
| Stoffstrom 1 (Rohneodekansäure) | 100 | Gewichtsteile/h |
| Stoffstrom 2 | 0,03 | Gewichtsteile/h |
| Stoffstrom 3 | 18,6 | Gewichtsteile/h |
| Stoffstrom 4 | 62,8 | Gewichtsteile/h |
| Stoffstrom 5 | 81,4 | Gewichtsteile/h |
| Stoffstrom 6 | 0,4 | Gewichtsteile/h |
| Stoffstrom 7 | 81,0 | Gewichtsteile/h |
| Stoffstrom 8 | 71,2 | Gewichtsteile/h |
| Stoffstrom 9 | 9,8 | Gewichtsteile/h |

Die Tabelle 2 zeigt die chemische Zusammensetzung der einzelnen Stoffströme in Gew.-%, die durch gaschromatographische Analysen ermittelt wurde.

Wie aus Tabelle 2 ersichtlich ist, trennt sich das in der Kolonne "A" über Kopf abgezogene azeotrope Gemisch in die beiden flüssigen Phasen (Stoffströme) 3 und 4, wobei mit der leichteren Phase 3 die Nebenprodukte fast vollständig ausgeschleust werden und die hauptsächlich das Schleppmittel Butandiol-1,3 enthaltende schwerere Phase 4 in die Kolonne "A" zurückgeführt wird.

In Kolonne "B" werden noch geringe Mengen an Zwischensiedern, hauptsächlich Carbonsäuren mit einer kleineren Kohlenstoffzahl als 10, abgetrennt.

Falls man einen geringen Gehalt dieser niederen Carbonsäuren in der Reinneodekansäure toleriert und an die Farbzahl keine höheren Ansprüche stellt, so kann auf die Kolonne "B" verzichtet werden.

In der Kolonne "C" wird die Neodekansäure als Kopfprodukt (Stoffstrom 8) mit 99,89 Gew.-%iger Reinheit und mit ausgezeichneter Farbzahl (APHA: 5-10) gewonnen.

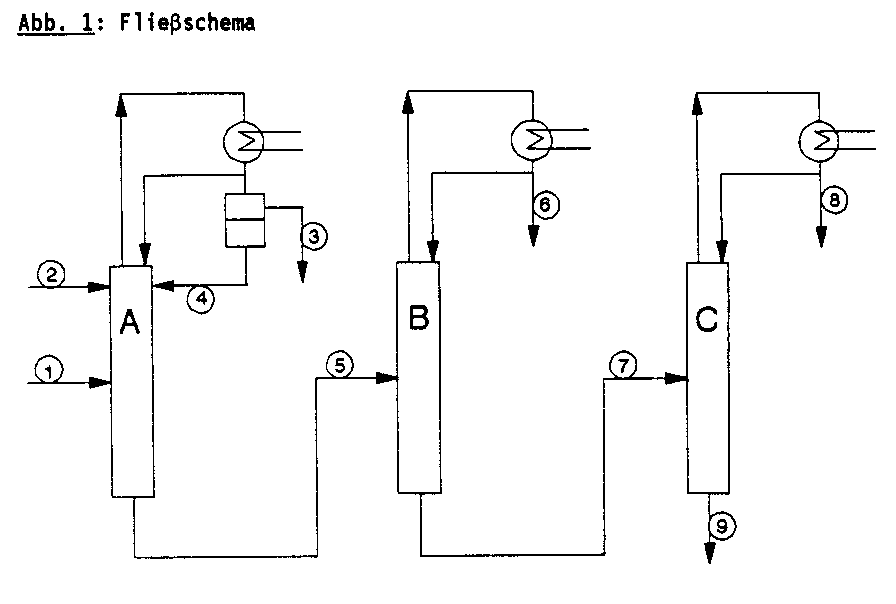

**Tabelle 1**

| **Verfahrensparameter** | | | |
|---|---|---|---|
| | **Kolonne "A"** | **Kolonne "B"** | **Kolonne "C"** |
| Kolonneneinbauten | 30 prakt. Böden | 6 m Gewebepackung | 5 m Gewebepackung |
| Produkt-Zulauf | 10. Boden von unten | 4 m Höhe von unten | 1 m Höhe von unten |
| Schleppmittel-Zulauf | Kolonnenkopf | ― | ― |
| Druck Kolonnenkopf | 10 mbar | 30 mbar | 10 mbar |
| Temp. Kolonnenkopf | 93 °C | 142 °C | 134 °C |
| Temp. Kolonnensumpf | 168 °C | 169 °C | 166 °C |
| Rücklaufverhältnis | 1 | 50 | 4 |

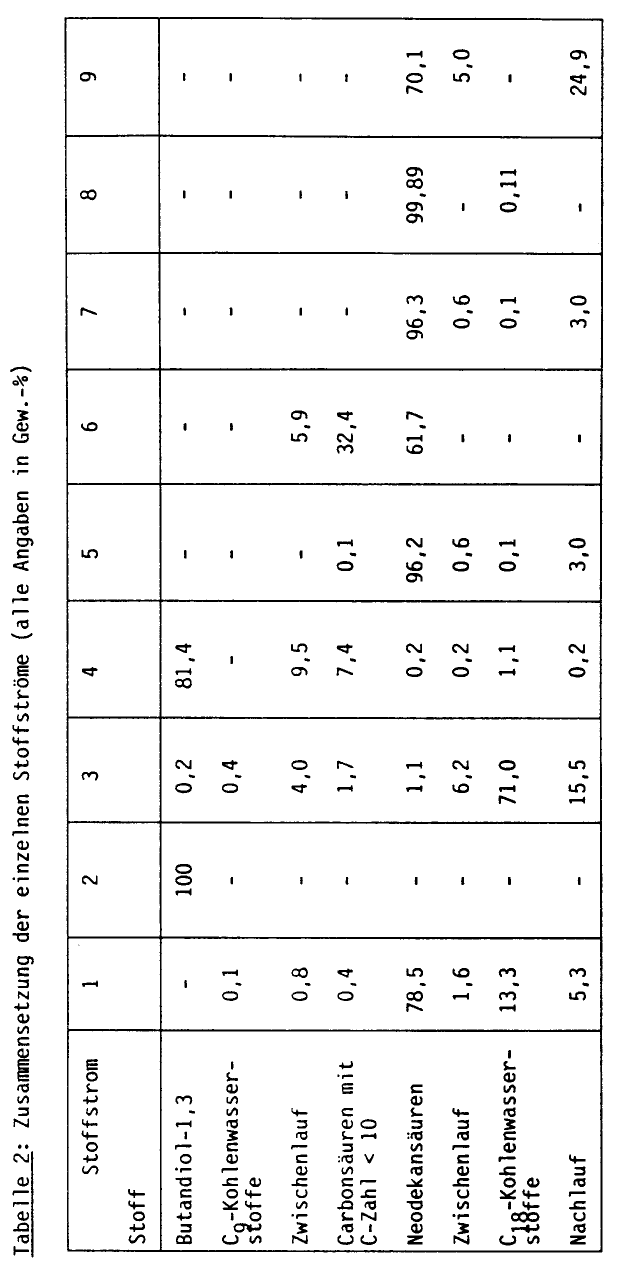

## Patentansprüche

1. Verfahren zur Herstellung von tertiären Carbonsäuren aus Kohlenmonoxid, den entsprechenden Olefinen und Wasser an einem sauren Katalysator,
dadurch gekennzeichnet,
daß die Nebenprodukte aus dem Reaktionsgemisch durch Azeotroprektifikation mit Alkandiolen als Schleppmittel nach Abtrennung des Katalysators entfernt und die tertiären Carbonsäuren gewonnen werden.

2. Verfahren nach Anspruch 1,
dadurch gekennzeichnet,
daß die Nebenprodukte, die ähnliche Siedepunkte wie die hergestellten tertiären Carbonsäuren aufweisen, aus dem Reaktionsgemisch durch Azeotroprektifikation mit Alkandiolen als Schleppmittel nach Abtrennung des Katalysators entfernt und die tertiären Carbonsäuren gewonnen werden.

3. Verfahren nach den Ansprüchen 1 und 2,
dadurch gekennzeichnet,
daß vor der Azeotroprektifikation mit Alkandiolen als Schleppmittel die nicht umgesetzten Einsatzolefine aus dem Reaktionsgemisch abgetrennt werden.

4. Verfahren nach Anspruch 3,
dadurch gekennzeichnet,
daß die nicht umgesetzten Einsatzolefine nach Abtrennung aus dem Reaktionsgemisch in den Reaktionsteil zurückgeführt werden.

5. Verfahren nach den Ansprüchen 1 bis 4,
dadurch gekennzeichnet,
daß die tertiären Carbonsäuren 6 bis 13 Kohlenstoffatome besitzen.

6. Verfahren nach Anspruch 5,
dadurch gekennzeichnet,
daß die tertiären Carbonsäuren 9 bis 10 Kohlenstoffatome besitzen.

7. Verfahren nach den Ansprüchen 1 bis 6,
dadurch gekennzeichnet,
daß bei der Herstellung von tertiären Carbonsäuren als Nebenprodukte gebildete Oligomere der Einsatzolefine und/oder Alkohole und/oder Ester und/oder Carbonylverbindungen und/oder aus den Einsatzstoffen stammende Paraffine und/oder Olefine durch Azeotroprektifikation mit Alkandiolen als Schleppmittel entfernt werden.

8. Verfahren nach Anspruch 7,
dadurch gekennzeichnet,
daß bei der Azeotroprektifikation als Schleppmittel Alkandiole eingesetzt werden, die mit den bei der Herstellung von tertiären Carbonsäuren als Nebenprodukte gebildeten Oligomeren der Einsatzolefine und/oder Alkoholen und/oder Estern und/oder Carbonylverbindungen und/oder aus den Einsatzstoffen stammenden Paraffinen und/oder Olefinen Heteroazeotrope bilden.

9. Verfahren nach den Ansprüchen 1 bis 8,
dadurch gekennzeichnet,
daß zur Herstellung von tertiären Nonansäuren und/oder tertiären Dekansäuren bei der Azeotroprektifikation als Schleppmittel Alkandiole mit Siedepunkten unter Normaldruck von < 230 °C eingesetzt werden.

10. Verfahren nach Anspruch 9,
dadurch gekennzeichnet,
daß bei der Azeotroprektifikation als Schleppmittel Butandiol-1,3 eingesetzt wird.

11. Verfahren nach den Ansprüchen 1 bis 10,
dadurch gekennzeichnet,
daß die Azeotroprektifikation mit Alkandiolen als Schleppmittel unter gegenüber Atmosphärendruck vermindertem Druck durchgeführt wird.

## Claims

1. Process for the production of tertiary carboxylic acids from carbon monoxide, the corresponding olefins and water on an acid catalyst,
characterized in that,
after separating off the catalyst, the by-products are removed from the reaction mixture by azeotropic rectification with alkanediols as carriers and the tertiary carboxylic acids are obtained.

2. Process according to claim 1,
characterized in that,
after separating off the catalyst, the by-products which have similar boiling points to the produced tertiary carboxylic acids are removed from the reaction mixture by azeotropic rectification with alkanediols as carriers and the tertiary carboxylic acids are obtained.

3. Process according to claims 1 and 2,
characterized in that
prior to the azeotropic rectification with alkanediols as carriers, the non-converted feed olefins are separated off from the reaction mixture.

4. Process according to claim 3,
characterized in that
the non-converted feed olefins, after separating off from the reaction mixture, are reintroduced into the reaction section.

5. Process according to claims 1 to 4,
characterized in that
the tertiary carboxylic acids have 6 to 13 carbon atoms.

6. Process according to claim 5,
characterized in that
the tertiary carboxylic acids have 9 to 10 carbon atoms.

7. Process according to claims 1 to 6,
characterized in that
in the production of tertiary carboxylic acids, oligomers of the feed olefins and/or alcohols and/or esters and/or carbonyl compounds and/or paraffins originating from the feed materials and/or olefins, formed as by-products, are removed by azeotropic rectification using alkanediols as carriers.

8. Process according to claim 7,
characterized in that
in the azeotropic rectification, alkanediols which form heteroazeotropes with the oligomers of the feed olefins and/or alcohols and/or esters and/or carbonyl compounds and/or paraffins originating from the feed materials, formed as by-products in the production of tertiary carboxylic acids, are used as carriers.

9. Process according to claims 1 to 8,
characterized in that
alkanediols with boiling points under normal pressure of < 230°C are used as carriers for the production of tertiary nonanoic acids and/or tertiary decanoic acids.

10. Process according to claim 9,
characterized in that
butanediol-1,3 is used as carrier in the azeotropic rectification.

11. Process according to claims 1 to 10,
characterized in that
the azeotropic rectification with alkanediols as carriers is carried out under pressure which is reduced vis-à-vis atmospheric pressure.

## Revendications

1. Procédé d'obtention d'acides carboxyliques tertiaires à partir d'oxyde de carbone, des oléfines correspondantes et d'eau sur un catalyseur acide, caractérisé en ce que les sous-produits provenant du mélange réactionnel sont éliminés par rectification azéotrope avec des alcanediols comme agent d'entraînement après séparation du catalyseur et en ce que les acides carboxyliques tertiaires sont isolés.

2. Procédé selon la revendication 1, caractérisé en ce que les sous-produits qui possèdent des points d'ébullition semblables à ceux des acides carboxyliques tertiaires produits, sont éliminés du mélange réactionnel par rectification azéotrope avec des alcanediols comme agents d'entraînement après séparation du catalyseur et en ce que les acides carboxyliques tertiaires sont isolés.

3. Procédé selon les revendications 1 et 2, caractérisé en ce qu'avant la rectification azéotrope avec des alcanediols comme agents d'entraînement, on sépare les oléfines utilisées qui n'ont pas réagi, du mélange réactionnel.

4. Procédé selon la revendication 3, caractérisé en ce que les oléfines utilisées qui n'ont pas réagi, sont séparées du mélange réactionnel.

5. Procédé selon les revendications 1 à 4, caractérisé en ce que les acides carboxyliques tertiaires possèdent de 6 à 13 atomes de carbone.

6. Procédé selon la revendication 5, caractérisé en ce que les acides carboxyliques tertiaires possèdent de 9 à 10 atomes de carbone.

7. Procédé selon les revendications 1 à 6, caractérisé en ce que lors de l'obtention d'acides carboxyliques tertiaires les oligomères formés comme sous-produits des oléfines utilisées et/ou des alcools et/ou des esters, et/ou des liaisons carbonyles et/ou des paraffines qui proviennent des substances d'utilisation et/ou des oléfines sont éliminés par rectification azéotrope avec des alcanediols comme agent d'entraînement.

8. Procédé selon la revendication 7, caractérisé en ce que lors de la rectification azéotrope on met en oeuvre des alcanediols qui forment avec les oligomères formés comme sous-produits lors de la production d'acides carboxyliques tertiaires des hétéroazéotropes des oléfines d'utilisation et/ou des alcools et/ou des esters et/ou des liaisons carbonyles et/ou des paraffines qui proviennent des substances d'utilisation et/ou des oléfines.

9. Procédé selon les revendications 1 à 8, caractérisé en ce que pour la production des acides nonanoïques tertiaires et/ou des acides décanoïques tertiaires lors de la rectification azéotrope on met en oeuvre comme agents d'entraînement des alcanediols ayant des points d'ébullition sous pression normale de < 230°C.

10. Procédé selon la revendication 9, caractérisé en ce que l'on met en oeuvre lors de la rectification azéotrope comme agent d'entraînement du butane 1,3-diol.

11. Procédé selon les revendications 1 à 10, caractérisé en ce que l'on effectue la rectification azéotrope avec des alcanediols comme agents d'entraînement sous pression réduite vis-à-vis de la pression atmosphérique.
